# EUROPEAN PATENT APPLICATION

(11) **EP 4 104 866 A1**
(43) Date of publication of application: **21.12.2022**
(21) Application number: 20918323.5
(22) Date of filing: 20.11.2020
(51) Int. Cl.: A61K 48/00, A61K 9/08, A61K 47/42, A61K 47/26, A61K 47/18, A61K 47/54

(54) **STABILIZER FOR ADENO-ASSOCIATED VIRUSES AND METHOD FOR STABILIZING ADENO-ASSOCIATED VIRUSES BY USING SAME**

(30) Priority: 10.02.2020 KR 20200015744
(71) Applicant: Innotherapy Inc., Seoul 07282 (KR)
(72) Inventor: KIM, Hong Kee, Goyang-si Gyeonggi-do 10230 (KR); LEE, Moon Sue, Seoul 06544 (KR); DO, Min Jae, Pohang-si Gyeongsangbuk-do 37562 (KR)
(74) Representative: Ostertag & Partner Patentanwälte mbB
(86) International application number: PCT/KR2020/016512
(87) International publication number: WO 2021/162211

(57) **Abstract**

Proposed are a stabilizer for an adeno-associated virus (AAV) and a method for stabilizing an adeno-associated virus using the same. More particularly, the present disclosure relates to a stabilizer for an adeno-associated virus including a surfactant or albumin, an adeno-associated virus liquid formulation including adeno-associated virus the stabilizer, and a production method for an adeno-associated virus with improved stability. The technology, according to the present disclosure, inhibits excessive aggregation between viruses when producing a gene delivery virus and therapeutic nanoparticles using an adeno-associated virus and forming an additional polyphenol binder for this purpose, thereby maintaining the particle size to an effective size for drug delivery and improving the stability in the liquid phase. Therefore, the technology is expected to be very useful in the field of drug delivery technology for gene therapy.

## Description

### Technical Field

The present disclosure relates to a stabilizer for an adeno-associated virus (AAV) and a method for stabilizing an adeno-associated virus using the same. More particularly, the present disclosure relates to a stabilizer for an adeno-associated virus including a surfactant or albumin, an adeno-associated virus liquid formulation including adeno-associated virus and the stabilizer, and a production method for an adeno-associated virus with improved stability.

### Background Art

Gene therapy is a technology to treat diseases by gene transfer and expression. Unlike drug therapy, gene therapy is a treatment to correct a genetic defect by targeting a specific gene that causes a disease. The ultimate goal of gene therapy is to obtain beneficial therapeutic effects by genetically modifying living cells. The main research field of gene therapy can be summarized as the field of introducing a gene that has a therapeutic effect on a specific disease, enhancing the resistance function of normal cells to show resistance to anticancer drugs, etc., or replacing a modified or lost gene in patients with various hereditary diseases.

Gene delivery technology for gene therapy can be divided largely into a method using a virus as a transporter (viral vector-based transfer method), a non-viral delivery method using synthetic phospholipids or cationic polymers, etc., and physical methods such as electroporation to introduce a gene by applying a temporary electrical stimulation to the cell membrane. Among the above delivery technologies, the viral vector-based transfer method uses a vector lacking some or all of the replication ability having a gene replaced with a therapeutic gene as a delivery medium, and is a preferred method for gene therapy because the delivery of the gene can be performed efficiently. Viruses used as viral carriers or viral vectors include RNA viral vectors (retroviral vectors, lentiviral vectors, etc.) and DNA viral vectors (adenoviral vectors, adeno-associated virus vectors, etc.), in addition, there are herpes simplex viral vectors, alpha viral vectors, and the like. Recently, studies on lentivirus and adeno-associated virus have been actively conducted (BRIC View 2016-T22) among the related studies.

Adeno-associated virus (AAV) is a single-stranded provirus belonging to the genus Parvoviridae and Dependovirus. The adeno-associated virus does not have the ability to proliferate alone and requires coexistence with auxiliary viruses such as Adenovirus and Vaccinia virus for replication. In the production of the adeno-associated virus, the adeno-associated virus is transfected with other plasmid DNAs expressing the rep part and the cap part, and the adenovirus is added as an auxiliary virus.

Adeno-associated virus is a very widely used vector in gene therapy because adeno-associated virus does not reproduce in infected cells, induces a relatively mild immune response, and the location of gene insertion is predictable to some extent. However, adeno-related viruses often produce genetic variants of wild-type protein shells to improve the production efficiency or infection efficiency and target delivery capabilities, and these virus surface changes may reduce stability in the external environment. In particular, since the recombinant adeno-associated virus is unstable in an aqueous solution, there are problems in aggregation and surface adsorption over time, and thus there has been a problem in long-term storage. In particular, this unstable property is a problem even when introducing functional molecules to the surface of the adeno-associated virus, and most of the adeno-associated viruses are aggregated during the introduction process. These aggregated viruses form crystals and grow to a size of 10 µm or more, thereby inducing embolism that blocks blood vessels and eventually causes the death of the injected individual.

The existing technology to improve the stability of adeno-associated viruses focused on how to store them for a long time without losing the infectivity of adeno-associated viruses as much as possible, but research to prevent crystallization caused when functional molecules are introduced to the surface has not progress much. Therefore, it is necessary to develop a technology for maintaining stability even when functional molecules are introduced while enhancing the stability of the adeno-associated virus itself.

### Disclosure

### Technical Problem

In the case of using adeno-associated virus (AAV) as a gene vector in the related art, aggregation and surface adsorption between AAVs occur due to the unstable characteristics of AAV in an in vivo/in vitro solution, resulting in poor stability. Moreover, when AAV is surface-modified with a material such as a polyphenol, excessive aggregation occurs, resulting in the formation of crystalline particles that may cause an embolism in the body. The present inventors have confirmed that aggregation and formation of crystalline particles between AAVs are remarkably suppressed by a surfactant or albumin treatment, and stability is maintained for a long period of time, thereby completing the present disclosure.

Accordingly, an objective of the present disclosure is to provide a stabilizer for adeno-associated virus (AAV), including a surfactant or albumin.

In addition, another objective of the present disclosure is to provide an adeno-associated virus liquid formulation including an adeno-associated virus (AAV) and a stabilizer being configured to include a surfactant or albumin.

In addition, still another objective of the present disclosure is to provide a production method for an adeno-associated virus with improved stability, the method includes treating an adeno-associated virus (AAV) with a stabilizer being configured to include a surfactant or albumin.

However, the technical problem to be achieved by the present disclosure is not limited to the above-mentioned problems, and other problems not mentioned will be clearly understood by those skilled in the art from the following description.

### Technical Solution

In order to achieve the objectives of the present disclosure as described above, the present disclosure provides a stabilizer for an adeno-associated virus (AAV), including a surfactant or albumin.

In one embodiment of the present disclosure, the surfactant may have a hydrophilic-lipophile balance (HLB) value of 5 to 20.

In another embodiment of the present disclosure, the surfactant may be selected from the group consisting of anionic, cationic, nonionic, and zwitterionic surfactants.

In still another embodiment of the present disclosure, the stabilizer may inhibit the aggregation of the adeno-associated virus.

In still another embodiment of the present disclosure, the adeno-associated virus may be surface-modified with a polyphenol-based material.

In still another embodiment of the present disclosure, the polyphenol-based material may be tannic acid, catecholamine, or epigallocatechin gallate (EGCG).

In still another embodiment of the present disclosure, the albumin may be included in a concentration of 10% to 0.01% (w/w).

In addition, the present disclosure also provides an adeno-associated virus liquid formulation, including an adeno-associated virus (AAV) and a stabilizer being configured to include a surfactant or albumin.

In one embodiment of the present disclosure, the liquid formulation may be maintained at a pH of 3.0 to 8.0.

In addition, the present disclosure also provides a production method for an adeno-associated virus with improved stability, the method includes treating an adeno-associated virus (AAV) with a stabilizer being configured to include a surfactant or albumin.

### Advantageous Effects

According to the present disclosure, the adeno-associated virus (AAV) for gene transfer is unstable by itself and easily aggregates between viruses. Moreover, when the surface is modified with polyphenol to provide additional functionality, excessive aggregation occurs between AAVs, and crystalline particles that can cause embolism, etc., may be formed. However, when a surfactant and/or albumin are treated together for surface modification, such aggregation between AAVs is significantly inhibited so that crystalline particles are not formed, and thus the in vitro stability of AAVs is enhanced. It was confirmed that a significant synergistic effect appeared when the surfactant and albumin were treated in combination. Therefore, the technology, according to the present disclosure, inhibits excessive aggregation between viruses when producing a gene transfer virus and therapeutic nanoparticles using an adeno-associated virus and forming an additional polyphenol conjugate for this purpose, thereby maintaining the particle size to an effective size for drug delivery and improving the stability in the liquid phase. Therefore, it is expected to be very useful in the field of drug delivery technology for gene therapy.

### Description of Drawings

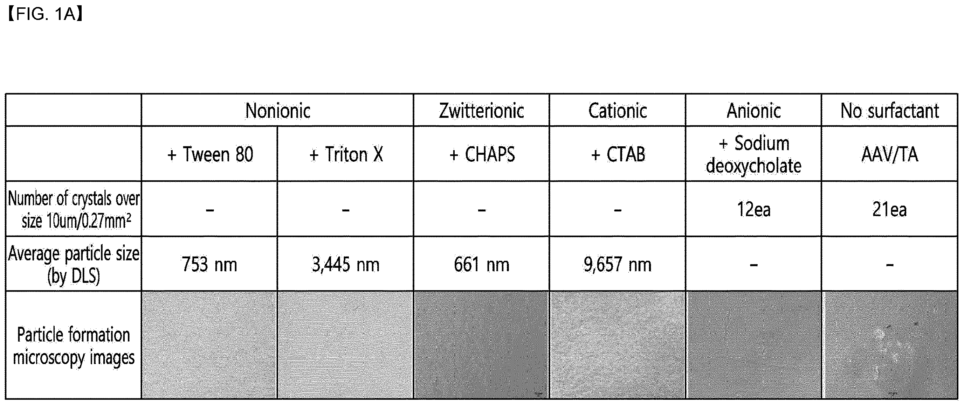

FIG. 1A is the result of observing crystal formation under a microscope and an average particle size measurement through the dynamic light scattering method after mixing the AAV solution treated with each of a nonionic surfactant of Tween 80 and Triton X, a zwitterionic surfactant of CHAPS, a cationic surfactant of CTAB, and an anionic surfactant of sodium deoxycholate, respectively, and tannic acid solution to verify the stability change of the adeno-associated virus (AAV) according to the type of surfactant;

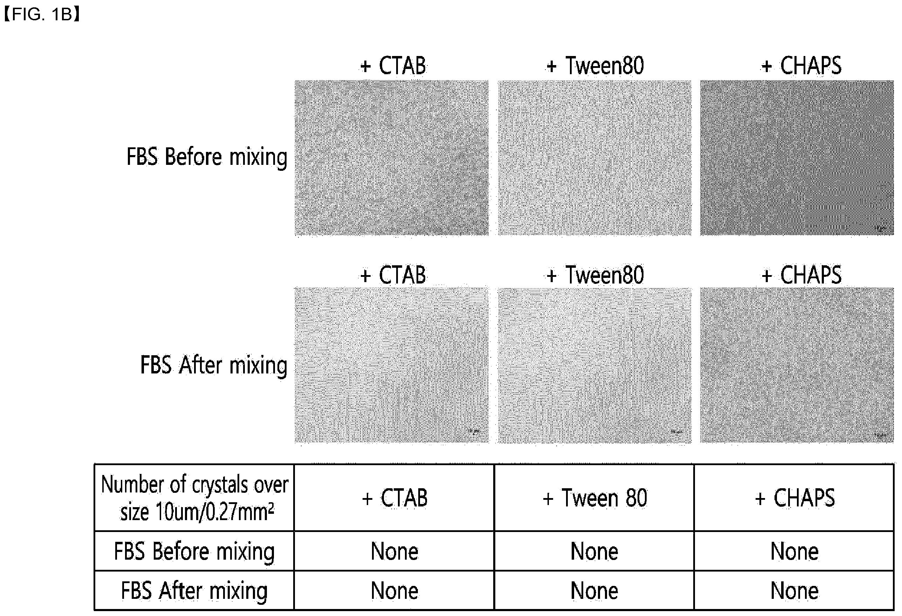

FIG. 1B is the result of confirming the formation of potential crystalline particles by serum mixing by mixing the AAV solution containing each of CTAB, Tween 80, and CHAPS with the tannic acid solution and then mixing FBS to observe whether crystals are formed;

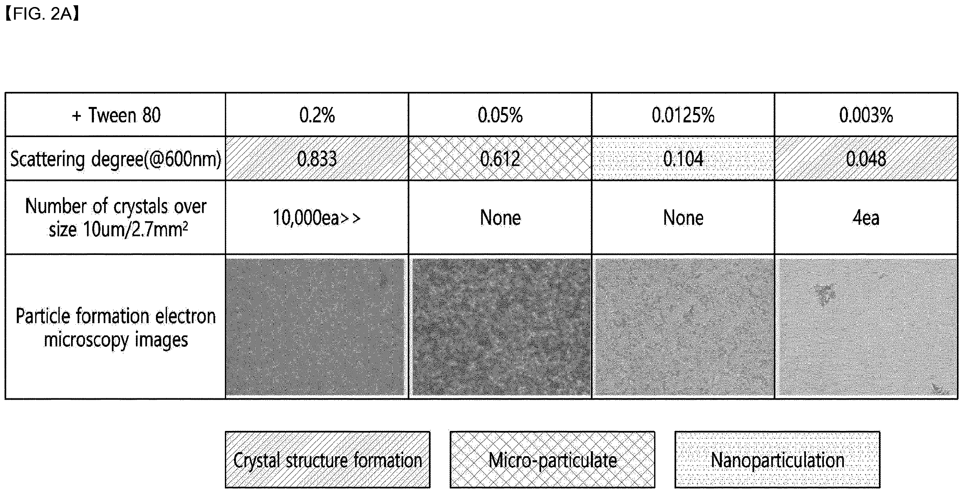

FIG. 2A is the result of measuring UV absorbance for scattering measurement after mixing with the AAV solution treated with Tween 80 in a concentration of 0.2%, 0.05%, 0.0125%, and 0.003% concentration, respectively, and tannic acid solution to verify the stability change of AAV according to the treatment concentration of each surfactant;

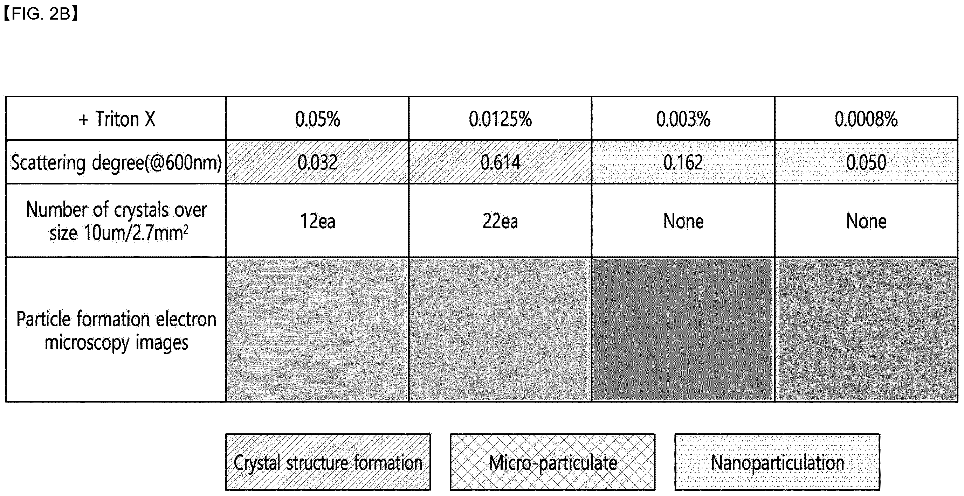

FIG. 2B is the result of measuring UV absorbance for scattering measurement after mixing the AAV solution treated with Triton X in a concentration of 0.05%, 0.0125%, 0.003%, and 0.0008%, respectively, and tannic acid solution;

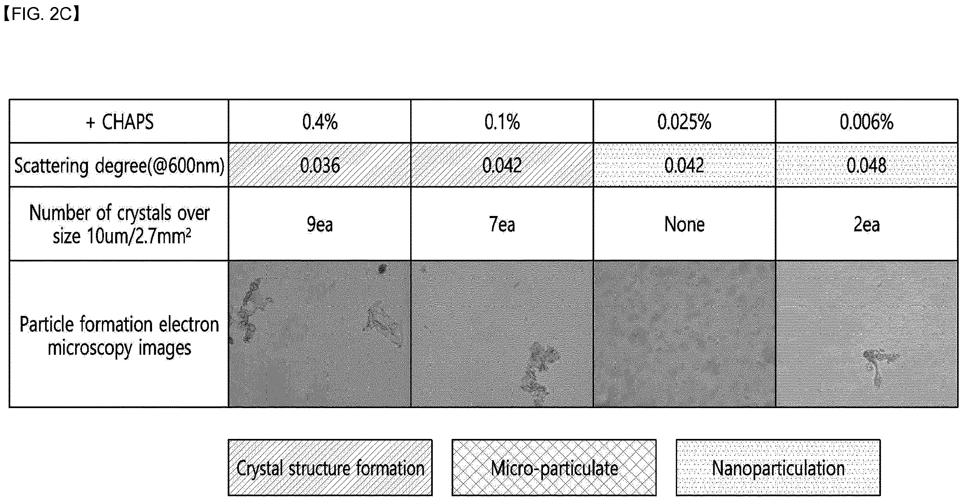

FIG. 2C is the result of measuring UV absorbance for scattering measurement after mixing the AAV solution treated with CHAPS in a concentration of 0.4%, 0.1%, 0.025%, and 0.006%, respectively, and tannic acid solution;

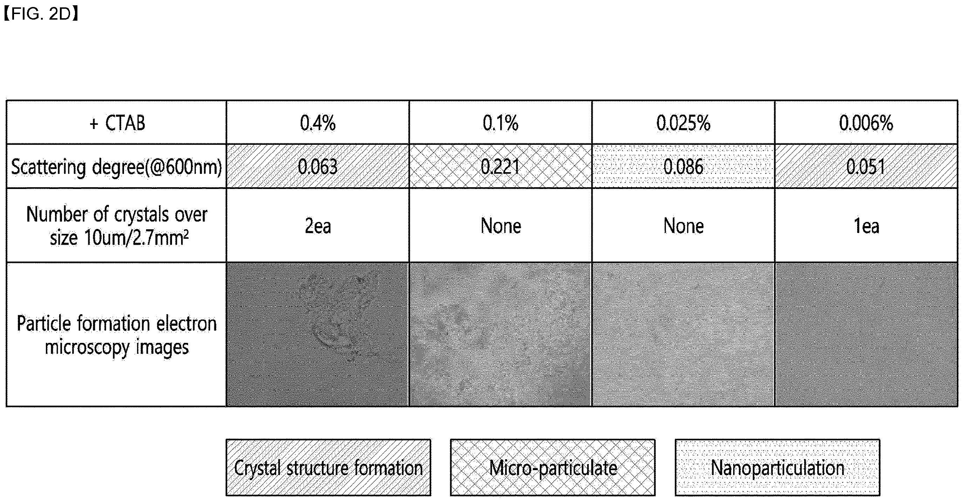

FIG. 2D is the result of measuring UV absorbance for scattering measurement after mixing the AAV solution treated with CTAB in a concentration of 0.4%, 0.1%, 0.025%, and 0.006%, respectively, and tannic acid solution;

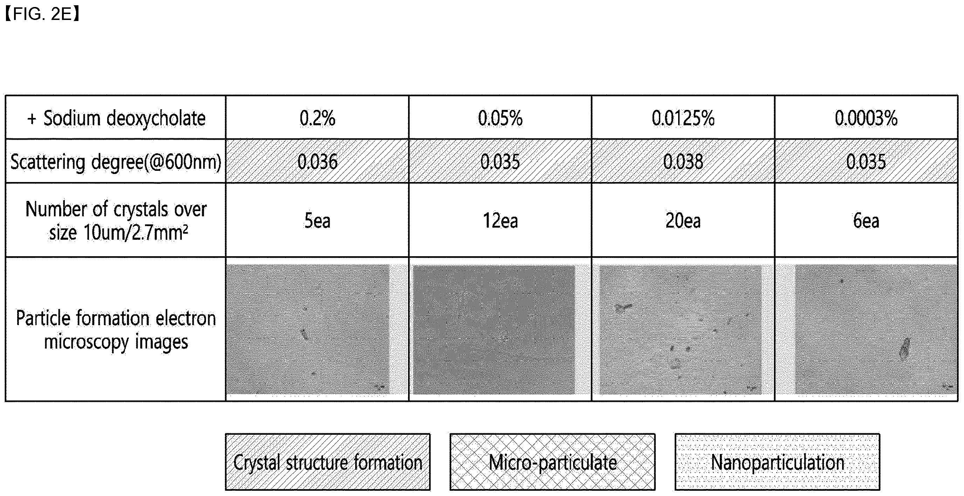

FIG. 2E is the result of measuring UV absorbance for scattering measurement after mixing the AAV solution treated with sodium deoxycholate in a concentration of 0.2%, 0.05%, 0.0125%, and 0.0003%, respectively, and tannic acid solution;

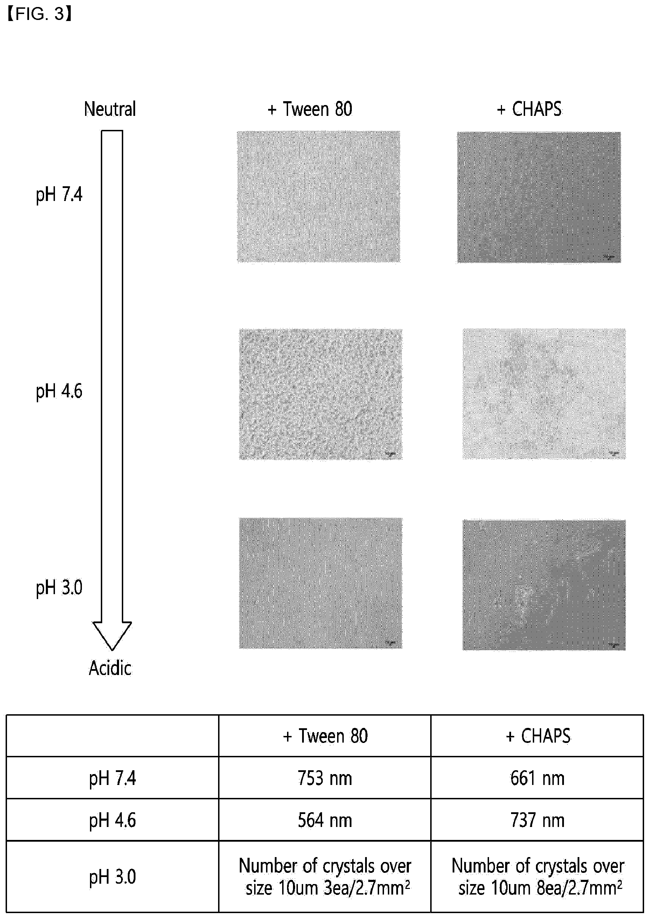

FIG. 3 is a result of verifying the stability change of AAV according to the pH conditions and is the result of measuring average particle size and observing the formation of crystalline particles after preparing an AAV solution in which Tween 80 and CHAPS are respectively present using buffer solutions having different pHs, mixing them with a tannic acid solution;

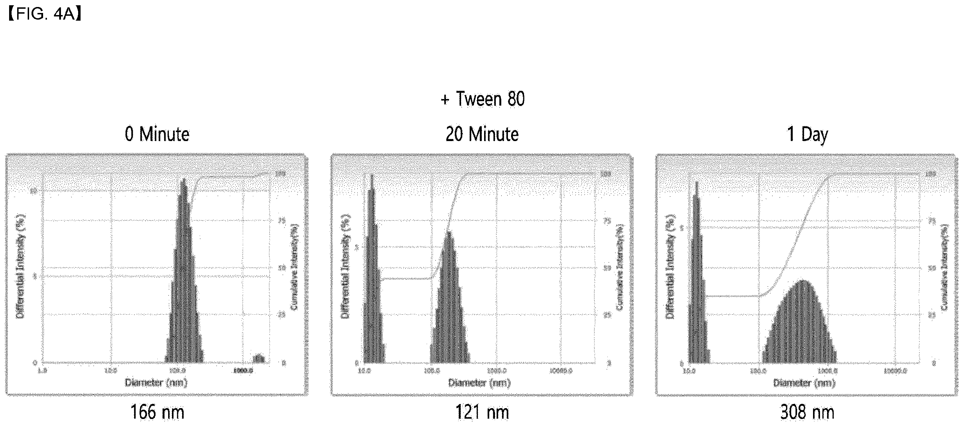

FIG. 4A is the result of measuring the average particle size after mixing the AAV solution treated with Tween 80 and tannic acid solution to verify the in vitro stability of AAV according to the surfactant treatment and is a result of observing the formation of crystalline particles and measuring the average particle size for 0 minutes, 20 minutes, and 1 day;

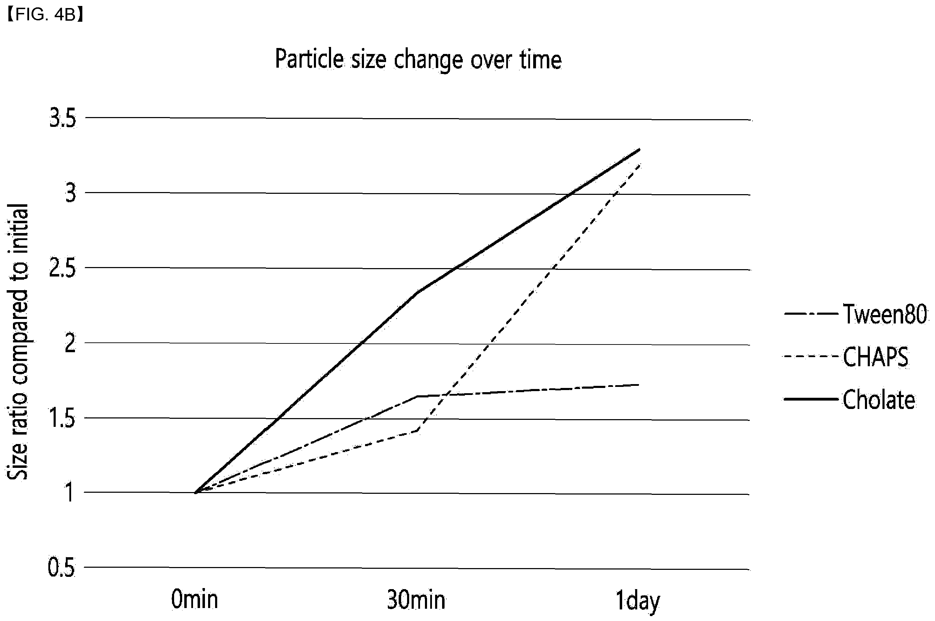

FIG. 4B is the result of measuring UV absorbance at 0 min, 30 min, and a day after mixing an AAV solution treated with Tween 80, CHAPS, and sodium deoxycholate, respectively, and tannic acid solution, and confirming a change in the size of particles over time through an increase in the degree of scattering compared to an initial value;

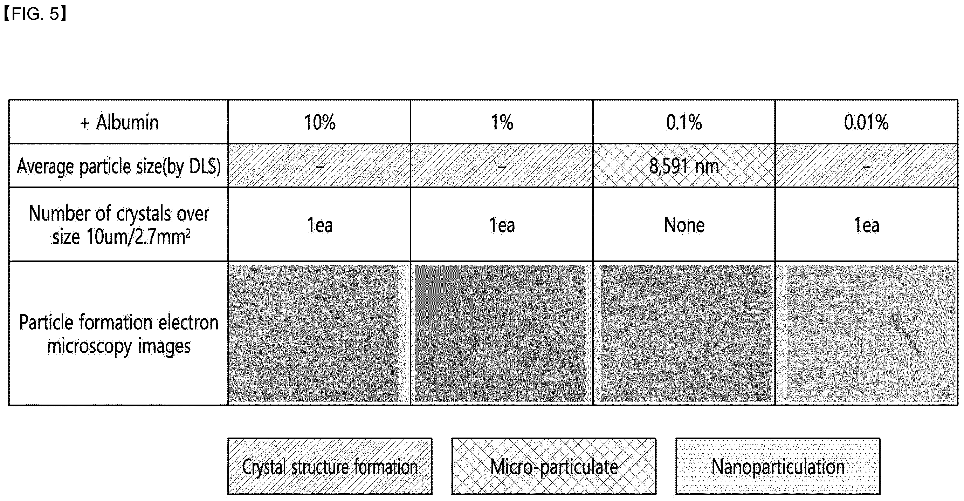

FIG. 5 is a result of verifying the stability enhancing effect of AAV according to the albumin treatment and is the result of measuring the average particle size and observing the formation of crystalline particles after mixing the AAV solution treated with albumin at various concentrations (10%, 1%, 0.1%, 0.01%) and the tannic acid solution;

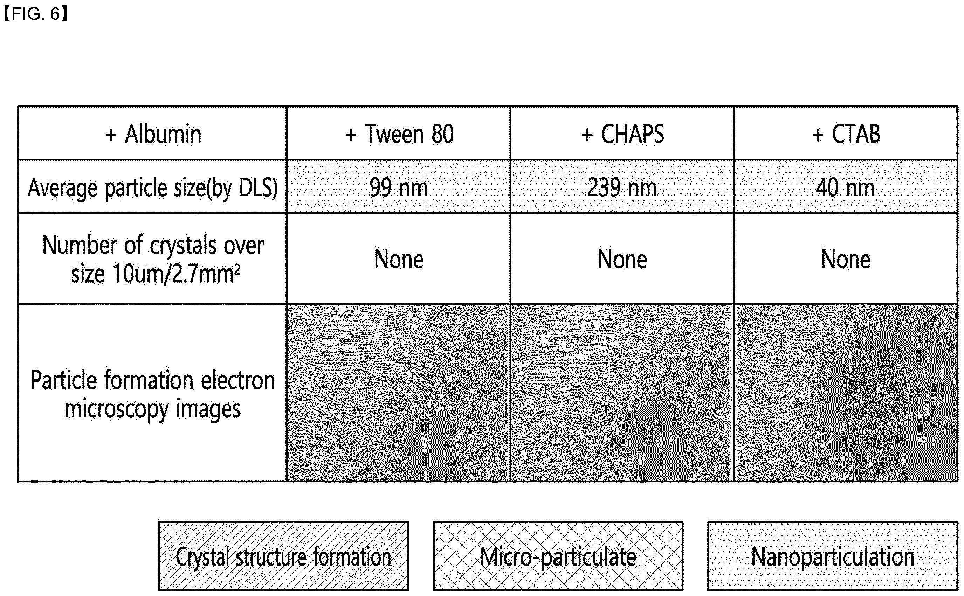

FIG. 6 is a result of verifying the effect of enhancing the stability of AAV by the combination of surfactant and albumin and is the result of measuring the average particle size and observing the formation of crystalline particles after mixing the AAV solution treated with each of the Tween 80, CHAPS, and CTAB and tannic acid solution, and then an albumin solution was additionally added; and

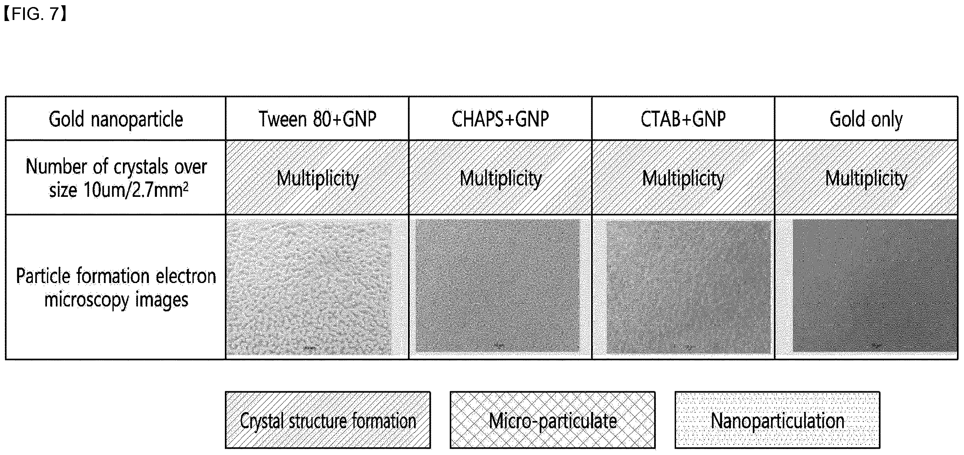

FIG. 7 is a result of verifying whether the stability of gold nanoparticles, which are inorganic nanoparticles other than AAV, is also maintained through the binding effect between the surfactant and tannic acid and is the result of measuring the average particle size and observing the formation of crystalline particles after mixing gold nanoparticles treated with each of Tween 80, CHAPS, and CTAB, and the tannic acid solution.

### Best Mode

The present inventors found that when adeno-associated virus (AAV) is used as a gene vector, aggregation and surface adsorption between AAVs occur due to the unstable characteristics of AAVs in an in vitro solution, resulting in poor stability. Moreover, when AAV is surface-modified with a material such as a polyphenol, excessive aggregation occurs, resulting in formation of crystalline particles that may cause embolism in the body. The present inventors have confirmed that aggregation and the formation of crystalline particles between AAVs are remarkably suppressed by a surfactant or albumin treatment and stability is maintained for a long period of time, thereby completing the present disclosure.

Accordingly, the present disclosure provides a stabilizer for adeno-associated virus (AAV) including a surfactant or albumin.

As used herein, the term stabilizer refers to a material added to prevent state change or chemical change when a material is produced, left alone, or preserved. In the present disclosure, the stabilizer may inhibit the aggregation between AAVs and may inhibit the formation of crystalline particles due to excessive aggregation when surface-modifying with a material such as a polyphenol. Furthermore, the stabilizer may have a stabilizing function so that it can be maintained the size and shape of the AAV in an aqueous solution for a long period of time until administered to the body.

In the present disclosure, a surfactant (detergent) is a material that reduces the surface tension by adsorbing to the interface in a dilute solution. Usually, amphiphilic material in which both a lipophilic group and a hydrophilic group are contained in one molecule may be a surfactant. In the present disclosure, the surfactant may include a hydrophilic-lipophile balance (HLB) value of 5 to 20. More preferably, the surfactant may be selected from the group consisting of anionic, cationic, nonionic, and zwitterionic surfactants.

The HLB value is a measure indicating the degree of hydrophilicity and lipophilicity of the surfactant, and when calculated through Griffin's formula for nonionic surfactants developed in 1954, HLB values between 0 and 20 are obtained, and an HLB value of 0 means a completely lipophilic/hydrophobic molecule, and an HLB value of 20 means a completely hydrophilic/oleophobic molecule. Through the HLB value, it is possible to infer the properties of the surfactant molecule to some extent.

The anionic surfactant refers to a surfactant in which an atomic group exhibiting surface activity becomes an anion by ionization when dissolved in water. The anionic surfactant has various types, and they can be broadly classified into soap, sulfuric acid ester type and alkylaryl sulfonate type of higher alcohol. In the present disclosure, sodium deoxycholate is used, but the usable type is not limited thereto. In the present disclosure, the anionic surfactant may be included in a concentration range of 0.1% to 0.01% (w/w) of the total solution and, preferably, may be included in a concentration of 0.07% to 0.03% (w/w), but is not limited thereto.

The cationic surfactant refers to a surfactant in which an atomic group exhibiting surface activity becomes a cation by ionization when dissolved in water. The cationic surfactant has hydrophilic and lipophilic atomic groups and is used in various fields such as powerful disinfectants, disinfectants, sedimentation accelerators, and antistatic agents. Most of them are nitrogen compounds (amine salts, quaternary ammonium salts), but there are also tertiary sulfonium salts or quaternary sulfonium salts of sulfur, and in the present disclosure, cetrimonium bromide (CTAB) is used but is not limited thereto. In the present disclosure, the cationic surfactant may be included in a concentration of 0.3% to 0.007% (w/w) of the total solution, preferably may be included in a concentration of 0.2% to 0.01% (w/w), more preferably may be included 0.1% to 0.02% (w/w), but is not limited thereto.

The nonionic surfactant is a surfactant that has polarity but does not separate into ions even when dissolved in water and has several weak hydrophilic groups and thus the hydrophilicity, that is, HLB, of the surfactant, varies according to the number of hydrophilic groups. The nonionic surfactant has high bubble stability, a harmless reaction to the skin, improved stability at low temperatures, excellent cleaning action, and less foaming, so its use is gradually expanding. The nonionic surfactant can be largely classified into ethylene oxide-based, diethanolaminebased, sorbitol-based, and glycerine-based, and Tween 80 and Triton X were used in the present disclosure but are not limited thereto. In the present disclosure, the nonionic surfactant may be included in a concentration range of 0.05 to 0.0001% (w/w) of the total solution, preferably may be included in a concentration of 0.04% to 0.0005% (w/w), more preferably may be included in a concentration of 0.03% to 0.0008% (w/w), and furthermore preferably may be included in a concentration of 0.02% to 0.001% (w/w), but is not limited thereto.

The zwitterionic surfactant is a surfactant having both a cationic atomic group and an anionic atomic group in a molecule, and there are organic compounds having various amphoteric electrolyte structures, so it is used as a shampoo, conditioner, softener, disinfectant, and the like. Various types of zwitterionic surfactants are known, and in the present disclosure, CHAPS (3-[(3-cholamidopropyl)dimethylammonio]-1-propanesulfonate) was used, but the type is not limited thereto. In the present disclosure, the zwitterionic surfactant may be included in a concentration range of 0.08% to 0.005% (w/w) of the total solution, preferably may be included in a concentration range of 0.06% to 0.01% (w/w), more preferably may be included in a concentration of 0.04% to 0.02% (w/w), but is not limited thereto.

Albumin is a simple protein widely distributed in living cells or body fluids, constitutes the basic material of cells together with globulin, and is widely present in the tissues of animals and plants. Albumin is an amphiphilic material such as a surfactant with hydrophilic and hydrophobic sites, so it has been widely used in biotechnology to dissolve poorly soluble drugs in water. In the present disclosure, albumin may be included in a concentration of 10% to 0.01% (w/w) of the total solution and preferably may be included in a concentration of 10% to 0.1% (w/w) but is not limited thereto.

The present inventors confirmed that the above surfactant effectively inhibited the aggregation of AAV and suppressed the formation of crystal particles through specific examples.

In one embodiment of the present disclosure, after mixing the AAV solution treated with different types of surfactants and the tannic acid solution, which is a surface modifying material of AAV, the average particle size is measured, and the formation of crystal particles is observed under a microscope. As a result, when the surfactant was not treated, a large number of crystalline particles were formed, whereas when the surfactant was treated, the aggregation of AAV was effectively inhibited so that the crystalline particles were not observed. It was confirmed that there was no significant effect even when injected into the mouse. In addition, it was confirmed that crystals were not formed regardless of FBS treatment as a result of additional verification by treating FBS to determine whether a potential crystal structure capable of blocking blood vessels was formed when mixed with plasma (see Example 2).

In another embodiment of the present disclosure, in order to find out the preferred concentration ratio between AAV, tannic acid, and surfactant, the stability of AAV was verified by treatment with various concentrations for each type of surfactant. Effective concentrations that do not form crystals were different depending on the type of surfactant, and where concentration was too high or too low, AAV aggregation was not effectively inhibited, and thus crystalline particles were formed (see Example 3).

In still another embodiment of the present disclosure, since the aspect of particle formation may vary depending on pH, AAV and tannic acid solutions containing surfactants were mixed with buffer solutions of various pHs, and the average particle size and crystalline particle formation were analyzed. As a result, the aspect of particle formation was changed before and after pH 3.0, and therefore, it was confirmed that the pH 3.0 to 8.0 range was preferably appropriate (see Example 4).

In still another embodiment of the present disclosure, in order to verify that the in vitro stability of AAV is maintained for a long period of time upon treatment with a surfactant, a surfactant-treated AAV and a tannic acid solution were mixed, and the particle size change was measured while leaving until 1 day later. As a result of the comparison, it was confirmed that the particle size was generally maintained until 1 day after, and when Tween 80 used as a nonionic surfactant was treated, the stability of AAV was maintained the highest (see Example 5).

In still another embodiment of the present disclosure, the effect of albumin as a material capable of improving the stability of AAV, such as a surfactant, was verified. To this end, after mixing the AAV solution and the tannic acid solution, the albumin was treated thereto at various concentrations (10%, 1%, 0.1%, 0.01%) to measure the average particle size and observe the formation of crystalline particles. As a result, it was confirmed that micro-sized particles were formed in the 0.1% concentration range (see Example 6-1). Furthermore, in order to investigate the effect of treatment with a surfactant, the AAV solution treated with each surfactant and the tannic acid solution were mixed, and the albumin solution was additionally mixed thereto, then the average particle size was measured, and the formation of crystalline particles was observed. As a result, the already formed particles were further reduced to a size of several hundred nanometers through albumin treatment, and the crystal structure was not formed, confirming the synergistic effect of the surfactant and albumin combination on the stability of AAV (Example 6-2 Reference).

In still another embodiment of the present disclosure, according to the results, it was verified whether the surfactant improves the stability of gold nanoparticles, which are inorganic nanoparticles other than AAV, and in the case of the gold nanoparticles, a crystal structure is formed in all cases regardless of the type of surfactant (see Example 7).

From the results of Examples of the present disclosure, it was found that various types of surfactants have different effective concentrations but have excellent effects in stabilizing AAV by inhibiting AAV aggregation and effectively inhibiting the formation of crystalline particles, and albumin also exhibits a more synergistic effect when combined with a surfactant.

Accordingly, as another aspect of the present disclosure, the present disclosure provides an adeno-associated virus liquid formulation configured to include an adeno-associated virus (AAV) and a stabilizer including a surfactant or albumin.

In the present disclosure, the liquid formulation may be maintained at a pH of 3.0 to 8.0.

In another aspect of the present disclosure, the present disclosure provides a production method for an adeno-associated virus with improved stability configured to include treating an adeno-associated virus (AAV) with a stabilizer including a surfactant or albumin.

In the present disclosure, a production method for an adeno-associated virus may use a method commonly used in the art, and a person skilled in the art may appropriately select and produce it.

The adeno-associated virus may be surface-modified with a polyphenol-based material in order to introduce cardiac targetoriented characteristics to the virus, and the polyphenol-based material is tannic acid, catecholamine, or epigallocatechin gallate (EGCG), preferably tannic acid, but is not limited thereto.

Hereinafter, preferred examples are presented to help the understanding of the present disclosure. However, the following examples are only provided for an easier understanding of the present disclosure, and the contents of the present disclosure are not limited by the following examples.

### [Example]

### Example 1. Experimental materials and methods

Tannic acid (T0200) and gold nanoparticles (900484) used in Examples of the present disclosure were purchased from Sigma-Aldrich, Co.

The gene recombinant adeno-associated virus serotype 9 (AAV 9) used in this Example was prepared through transient transfection.

Briefly, it was prepared by transfection of an AAV helper plasmid of the same mass having cap3.45, which is the capsid gene of AAV 9, a CMV GFP vector plasmid with an inverted terminal repeat (ITR), and an adenoviral helper plasmid into AAV-293 cells using calcium phosphate. Thereafter, the prepared viral vectors were recovered as described in the previous study, and impurities were removed by ultracentrifugation. The genomic titer of AAV was derived using QPCR.

### Example 2. Verification of AAV stability change according to surfactant type

The present inventors tried to check the change in AAV stability by treating a surfactant to solve the problem of AAV aggregation when preparing a gene delivery system using AAV. To this end, the effect was verified for various types of surfactants known in the art. More specifically, after mixing 50 uL of 0.5 mM tannic acid solution with 1x108 genome/ul AAV solution in which each of Tween 80, Triton X, CHAPS, cetrimonium bromide (CTAB), and sodium deoxycholate was present in a concentration of 0.05% (w/w) (PBS, pH 7.4), and then after about 10 minutes, the average particle size was measured through dynamic light scattering (DLS), and the formation of crystals that could cause blood vessel clogging was observed under a microscope. At this time, as a condition not treated with a surfactant, 50 uL of a 1x108 genome/ul AAV solution (PBS, pH 7.4) was mixed with 50 uL of 0.5 mM tannic acid solution, and average particle size measurement and microscopic observation were performed in the same manner as above.

As a result, as shown in FIG. 1A, when the surfactant was not present, it was observed that crystalline particles were formed in the form of tens to hundreds of micrometers, whereas surfactants such as Tween 80 and Triton X as nonionic surfactants, CHAPS as zwitterionic surfactants, and CTAB as cationic surfactants were treated, particles in the form of a crystalline form in which AAV was aggregated were not observed. In addition, when sodium deoxycholate, an anionic surfactant, was treated, large fibrous particles were observed, but it was confirmed that a better aspect appeared compared to the case where the surfactant was not treated.

On the other hand, the presence or absence of the formation of such crystalline particles is one of the major factors affecting the survival of experimental animals in animal experiments. Crystalline particles of more than 10 µm can block capillaries in the body, and clogged blood vessels can cause ischemic disease, myocardial infarction, cerebral infarction, rupture of blood vessels, etc., and in severe cases, leading to death. Therefore, the present inventors tried to experimentally confirm how the crystallinity was confirmed in the results of FIG. 1A affects the survival of mice. To this end, 50 uL of 0.5 mM or 0.25 mM tannic acid solution was mixed with 50 uL of 2x109 genome/ul AAV solution containing Tween 80 in a concentration of 0.01% (w/w) (PBS, pH 7.4), and then viability was confirmed by injecting 100 uL of mixed solution into the tail vein of the mouse within about 30minutes (0.01%(w/w) Tween 80 + 0.5 mM TA + AAV / 0.01% (w/w) Tween 80 + 0.25 mM TA + AAV). In addition, as a condition with the absence of surfactant, 50 uL of 2x109 genome/ul AAV solution without Tween 80 was mixed with tannic acid under the same conditions and injected into the tail vein of mice (0.5 mM TA + AAV / 0.25 mM TA + AAV). In order to compare the conditions under which only the virus was injected, 50 uL of a PBS solution was prepared in 50 uL of a 2x109 genome/ul AAV solution and injected under the same conditions (virus). In addition, 100 uL of 0.5 mM tannic acid solution (TA) and 100 uL of PBS (PBS) were respectively injected.

As a result, as shown in Table 1 below, when AAV was mixed with tannic acid and injected, the mouse died immediately regardless of the concentration of tannic acid. When only AAV or tannic acid was injected, the mouse did not die, but when AAV was mixed with tannic acid and injected, it was considered the crystal is formed by mixing, and when AAV is mixed with tannic acid under the conditions containing surfactants, the mouse survived. Therefore, it was determined that the role of the surfactant in inhibiting the formation of crystals is very important in suppressing severe blood vessel clogging.

**[Table 1]**

| **Condition** | **Result** |
|---|---|
| 0.01% Tween 80 + 0.5 mM TA + AAV | Survival (n=6) |
| 0.01% Tween 80 + 0.25 mM TA + AAV | Survival (n=5) |
| 0.5 mM TA + AAV | Death (n=1) |
| 0.25 mM TA + AAV | Death (n=1) |
| Virus | Survival (n=2) |
| PBS | Survival (n=2) |
| TA | Survival (n=2) |

Furthermore, the present inventors have attempted to verify whether the formation of a potential crystal structure that may block blood vessels when mixed with plasma may be suppressed through the treatment of a surfactant additionally. To this end, 50 uL of 0.5 mM tannic acid solution was mixed with 50 uL of 1x108 genome/ul AAV solution (PBS, pH 7.4) containing each of CTAB, Tween 80, and CHAPS in a concentration of 0.05% (w/w), and then about 5 minutes after, 50 uL of 10% FBS solution was mixed and observed whether crystals of 10 µm or larger were formed under a microscope. As a result, it was confirmed that even after mixing the FBS, as can be seen in FIG. 1B, the state in which crystals are not formed is maintained as before mixing.

### Example 3. Verification of AAV stability change according to surfactant concentration

From the result of Example 2, it was confirmed that when AAV and tannic acid were mixed by treating the surfactant together, it was possible to inhibit aggregation and formation of crystals. Therefore, in addition to this, in order to find out the preferred concentration ratio of the surfactant, the aspect of particle formation for each concentration of the surfactant was analyzed.

### 3-1. Nonionic surfactant

50 uL of 0.5 mM tannic acid solution was mixed with 1x108 genome/ul AAV solution in which Tween 80, a nonionic surfactant, is present in a concentration of 0.2%, 0.05%, 0.0125%, and 0.003% (w/w) (PBS, pH 7.4), respectively, and about 5 minutes after, UV-Vis absorbance in the 600 nm wavelength band was measured for scattering, and crystal formation was observed under a microscope.

In addition, under conditions in which Triton X, another nonionic surfactant, is present in a concentration of 0.05%, 0.0125, 0.003%, and 0.0008% (w/w), respectively, AAV and tannic acid solutions were mixed in the same method as above, and the same experiment was performed, and the result was observed.

As a result, as shown in FIG. 2A, when Tween 80 was included in a concentration of 0.2% and 0.003%, a crystal structure was formed, but when Tween 80 was included in 0.05% and 0.0125%, micro and nano-sized particles were formed, respectively. In addition, as shown in FIG. 2B, when Triton X was included in a concentration of 0.05% and 0.0125%, a crystal structure was formed, but when Triton X was included in a concentration of 0.003% and 0.0008%, nano-sized particles were formed.

### 3-2. Zwitterionic surfactant

50 uL of 0.5 mM tannic acid solution was mixed with 1x108 genome/ul AAV solution in which the zwitterionic surfactant CHAPS is present in a concentration of 0.4%, 0.1%, 0.025%, 0.006% (w/w) (PBS, pH 7.4), respectively, and after about 5 minutes, UV-Vis absorbance in the 600 nm wavelength band was measured for scattering, and crystal formation was observed under a microscope.

As a result, as shown in FIG. 2C, when CHAPS was included in a concentration of 0.4%, 0.1%, and 0.006%, a crystal structure was formed, but in the case of 0.025%, nano-sized particles were formed.

### 3-3. Cationic surfactant

50 uL of 0.5 mM tannic acid solution was mixed with 1x108 genome/ul AAV solution in which the cationic surfactant CTAB is present in a concentration of 0.4%, 0.1%, 0.025%, 0.006% (w/w) (PBS, pH 7.4), respectively, and after about 5 minutes, UV-Vis absorbance in the 600 nm wavelength band was measured for scattering, and crystal formation was observed under a microscope.

As a result, as shown in FIG. 2D, when CTAB was included in a concentration of 0.4% and 0.006%, a crystal structure was formed, but when CTAB was included in 0.1% and 0.025%, micro and nano-sized particles were formed, respectively.

### 3-4. Anionic surfactant

50 uL of 0.5 mM tannic acid solution was mixed with 1x108 genome/ul AAV solution in which the anionic surfactant sodium deoxycholate is present in a concentration of 0.2%, 0.05%, 0.0125%, 0.0003% (w/w) (PBS, pH 7.4), respectively, and after about 5 minutes, UV-Vis absorbance in the 600 nm wavelength band was measured for scattering, and crystal formation was observed under a microscope.

As a result, as shown in FIG. 2E, in the case of sodium deoxycholate, it was confirmed that the crystal structure was formed at all concentrations.

Through the above results, the effective concentration that does not form crystals is different depending on the type of surfactant, and when the concentration is too high or too low, the binding of tannic acid and AAV cannot be effectively controlled, and thus crystalline particles tend to be formed.

### Example 4. Verification of AAV stability change according to pH conditions

In the formation of micro or nano-sized particles, the formation aspect may be changed according to the influence of an external environment, and in particular, the aspect of particle formation may vary depending on pH conditions. Therefore, the present inventors tried to verify whether the stability of AAV changes according to pH conditions. To this end, 1x108 genome/ul AAV solution in which Tween 80 and CHAPS are present in a concentration of 0.05% (w/w) (PBS, pH 7.4) (PBS, pH 7.4) was prepared using different buffer solutions (PBS pH 7.4, citric acid pH 4.6, DDW pH 3.0), respectively. After 50 uL of a 0.5 mM tannic acid solution was mixed with the solution, the average particle size was measured through a dynamic light scattering method, and the formation of crystalline particles were observed under a microscope.

As a result, as shown in FIG. 3, it was confirmed that the size of the formed particles and the degree of crystalline form before and after pH 3.0 was changed. It was found that, under the conditions of pH 3.0 or less, excessive aggregation occurred, and crystalline particles were formed. In addition, in the case of polyphenols, since oxidation reactions are promoted under basic pH conditions, the preferred pH range was determined to be between about 3.0 and 8.0 in consideration of the long-term stability of the particles.

### Example 5. In vitro stability verification

The size and shape of AAV particles must be maintained constantly from mixing to injection. Therefore, it was analyzed whether the particle size was maintained until after 30 minutes, which is a time taken from mixing to injection, and a sufficient time, one day. To this end, 50 uL of 0.5 mM tannic acid solution was mixed with 1x108 genome/ul AAV solution in which Tween 80 is present in a concentration of 0.01% (w/w) (PBS, pH 7.4), and then, after about 5 minutes, the average particle sizes were measured at 0 min, 20 min, and 1 day, respectively, through a dynamic light scattering method, and the increase in particle sizes was compared with the initial value.

As a result, as shown in Fig. 4A, although there was a slight difference in the particle size until the 1st day, it was confirmed that the particle size was generally maintained after it was first made.

In addition, 50 uL of 0.5 mM tannic acid solution was mixed with 1x108 genome/ul AAV solution in which various types of surfactants, i.e., Tween 80, CHAPS, and sodium deoxycholate are present in a concentration of 0.05% (w/w) each (PBS, pH 7.4), and then, after about 5 minutes, UV-Vis absorbance of each 600 nm wavelength band was measured at 0 min (min), 30 min, and 1 day (day) to measure the scattering degree. The change in particle size over time was analyzed through an increase in scattering compared to the initial value.

As a result, it was confirmed that the particle size was most stably maintained in the condition in which Tween 80 was added when the size change of the particles formed by each surfactant was compared, as shown in FIG. 4B.

### Example 6. Verification of stability enhancement effect by albumin

The present inventors attempted to further reduce the size of the particles by treating additional additives in particle formation through bonding between polyphenols and surfactants. To this end, albumin was selected as the additive, and the effect thereof was verified.

### 6-1. Effect verification by albumin alone

50 uL of 0.5 mM tannic acid solution was mixed with 1x108 genome/ul AAV solution in which the albumin is present at various concentrations of 10%, 1%, 0.1%, 0.01% (w/w) (PBS, pH 7.4), respectively, and after about 10 minutes, the average particle size was measured through dynamic light scattering, and particle formation was observed under a microscope.

As a result, as can be seen in FIG. 5, when albumin was added in a concentration of 10%, 1%, and 0.01%, a crystal structure was formed, whereas in the case of 0.1% concentration, micro-sized particles were formed.

### 6-2. Verification of stability enhancement effect by combination of surfactant and albumin

Next, in order to find out whether albumin can further reduce the size of particles already formed through each surfactant, 50 uL of 0.5 mM tannic acid solution was mixed with 1x108 genome/ul AAV solution in which Tween 80 is present in a concentration of 0.01% (w/w), CHAPS and CTAB are present each in a concentration of 0.05% (w/w)(PBS, pH 7.4), and after about 5 minutes, 50 uL of 10% (w/w) albumin solution was mixed, and then the average particle size was measured through dynamic light scattering and observed under a microscope.

As a result, as shown in FIG. 6, it was confirmed that by adding albumin together with the surfactant, particles already formed through each surfactant were reduced to hundreds of nanometers through albumin treatment and a crystal structure of 10 um or more that may cause vascular clogging was not formed. From the above results, it was found that the stability of AAV was further improved by the combination of the surfactant and albumin.

### Example 7. AAV-specific stability effect verification

The present inventors conducted the following experiment to find out whether the stability of gold nanoparticles, which are inorganic nanoparticles that can be used as gene vectors other than AAV, is also maintained through the binding effect of surfactants and tannic acid. Specifically, 50 uL of 0.5 mM tannic acid solution was mixed with 50 uL of gold nanoparticle solution with 450 nm absorbance 0.1 AU concentration in which Tween 80, CHAPS, and CTAB were present in a concentration of 0.05% (w/w) (PBS, pH 7.4), respectively. After about 5 minutes, the average particle size was measured through dynamic light scattering and observed under a microscope. In addition, the experiment was conducted under the same conditions in which no surfactant was added as a control group.

As a result, as shown in FIG. 7, it was confirmed that in the case of gold nanoparticles, crystal structures were formed in all cases regardless of the type of surfactant, and more severe aggregation than AAV was observed due to the weight of the particles themselves. Through this, in the case of inorganic particles such as gold nanoparticles, it was judged that it was not appropriate to apply a technique for maintaining the stability of the particles by treating them with a surfactant.

The above-described description of the present disclosure is, for example, and it will be understood by those skilled in the art that the present disclosure may be easily modified in other specific forms without changing the technical spirit or essential features of the present disclosure. Therefore, it should be understood that the embodiments described above are exemplary and not limited in all respects.

### Industrial Applicability

The stabilization technology of the adeno-associated virus, according to the present disclosure, inhibits excessive aggregation between viruses when producing a gene delivery virus and therapeutic nanoparticles using an adeno-associated virus and forming an additional polyphenol binder for this. Since excessive aggregation between viruses is inhibited, the size of particles is maintained at an effective size for drug delivery, and stability in a liquid phase is increased. It may be useful in gene treatment and related fields using adenosine-related viruses.

## Claims

1. A stabilizer for an adeno-associated virus, the stabilizer comprising a surfactant or albumin.

2. The stabilizer of claim 1, wherein the surfactant has a hydrophilic-lipophile balance value of 5 to 20.

3. The stabilizer of claim 2, wherein the surfactant is selected from the group consisting of anionic, cationic, nonionic, and zwitterionic surfactants.

4. The stabilizer of claim 1, wherein the stabilizer inhibits the adeno-associated viruses from aggregating.

5. The stabilizer of claim 1, wherein the adeno-associated virus is surface-modified with a polyphenol-based material.

6. The stabilizer of claim 5, wherein the polyphenol-based material is tannic acid, catecholamine, or epigallocatechin gallate.

7. The stabilizer of claim 1, wherein the albumin is comprised in a concentration of 10% to 0.01% (w/w).

8. An adeno-associated virus liquid formulation comprising an adeno-associated virus and a stabilizer comprising a surfactant or albumin.

9. The liquid formulation of claim 8, the liquid formation is maintained at pH 3.0 to 8.0.

10. A method of preparing adeno-associated virus with improved stability, the method comprising treating an adeno-associated virus with a stabilizer,
wherein the stabilizer comprises a surfactant or albumin.

11. The method of claim 10, wherein the adeno-associated virus is surface-modified with a polyphenol-based material.

12. The method of claim 11, wherein the polyphenol-based material is tannic acid, catecholamine, or epigallocatechin gallate.
